# EUROPEAN PATENT SPECIFICATION

(11) **EP 4 390 960 B1**
(45) Date of publication and mention of the grant of the patent: **24.06.2026**
(21) Application number: 23209188.4
(22) Date of filing: 10.11.2023
(51) Int. Cl.: G16H 30/40, G16H 50/20, G16H 50/70, G16H 15/00

(54) **SYSTEMS AND METHODS FOR PROVIDING AN UPDATED MACHINE LEARNING ALGORITHM**
SYSTEME UND VERFAHREN ZUR BEREITSTELLUNG EINES AKTUALISIERTEN MASCHINENLERNALGORITHMUS
SYSTÈMES ET PROCÉDÉS POUR FOURNIR UN ALGORITHME D'APPRENTISSAGE AUTOMATIQUE MIS À JOUR

(30) Priority: 20.12.2022 DE 102022214014
(43) Date of publication of application: 26.06.2024
(73) Proprietor: Siemens Healthineers AG, 91301 Forchheim (DE)
(72) Inventor: Abdishektaei, Mohammad, Charlottesville, 22903 (US); Farhand, Sepehr, Malvern, 19355 (US); Hermosillo Valadez, Gerardo, West Chester, 19382 (US); Shinagawa, Yoshihisa, Downingtown, 19335 (US); Thesen, Stefan, 91077 Dormitz (DE); Vinay Bhombore, Vineet, 560078 Bengaluru (IN)
(74) Representative: Siemens Healthineers Patent Attorneys

(56) References cited:
- WO-A1-2020/142401
- US-A1- 2014 088 989
- US-A1- 2022 108 070

## Description

Independent of the grammatical term usage, individuals with male, female or other gender identities are included within the term.

The invention concerns the provision of updated machine learning algorithm, in particular, in a healthcare environment. Further, the invention concerns systems and methods in the field of federated learning as well as systems and methods for the automated provision of training data based on healthcare information, in particular, based on electronic medical records.

Advances in medical imaging, e.g., employing computed tomography or magnetic resonance systems, allow for reproducing tiniest changes in the anatomy of a patient. As a result of the increased performance of these systems, there is increased focus on the early detection of diseases with improved chances of success of the following treatment. However, for radiologists, this increased focus also has negative aspects. The procedure of visually analyzing radiology images is often challenging. For instance, the density and tissue type of organs are highly varied and in turn present a high variety of visual features. Additionally, background visual patterns can obscure the early signs of malignancies which may then be easily overlooked by the human eye. Therefore, the manual classification of the spatial distribution of abnormalities or patterns inevitably leads to errors owing to mistakes, human error, and/or details too fine for the human eye to detect. Thus, the analysis of medical images may lead to false negatives which may cause missed treatment. Likewise, the evaluation may prompt false positives which may cause unwanted psychological and sub-optimal downstream diagnostic and treatment consequences.

What is more, the reliable detection of abnormalities and/or features in medical images often requires highly experienced physicians further increasing their workload. Moreover, the human component in evaluating image data adds a degree of subjectivity which is often unwanted.

To cope with such problems, computer- aided detection (CADe) and computer-aided diagnosis (CADx) systems are being developed. Hereafter both types of systems will be referred to as CAD systems. CAD systems are technologies to help radiologists interpret medical images. A common use of CAD systems is to automatically identify suspicious regions in a medical image. Such suspicious regions may contain image patterns indicative of abnormalities which may comprise cancerous growths, masses, abscesses, lacerations, calcifications, lesions and/or other irregularities within biological tissue and which can cause serious medical problems if left undetected.

US 2014 / 0 088 989 A1 discloses learning a predictive model from routine patient data from multiple medical centers.

WO 2020 / 142 401 A1 discloses computer-implemented techniques for integrating artificial intelligence informatics into various systems, including healthcare systems, using a distributed learning platform.

To date, machine learning algorithms have proven highly effective in the automated detection of medical findings. One issue is that suchlike algorithms have to be trained on a sufficient amount of training data of sufficient quality to work properly during inference. Training data is particularly precious since it has to come with a ground truth which the machine learning algorithm can use for comparing its results and making adjustments in order to improve the performance. Oftentimes, the ground truth relies on expert annotations. That is, a human expert has to annotate medical findings in medical data sets manually. This is a tedious task especially if huge numbers of training data sets have to provided for training complex detection algorithms.

It is a task of the present invention to improve this situation by providing systems and methods capable of facilitating a more efficient usage medical data at a clinical set and a more efficient training of machine learning algorithms.

This object is solved by methods for clinical decision support, corresponding systems, a corresponding computer-program product and computer-readable storage medium according to one or more of the independent claims. Alternative and/or preferred embodiments are subject of the dependent claims.

In the following, the technical solution according to the present invention is described with respect to the claimed apparatuses as well as with respect to the claimed methods. Features, advantages or alternative embodiments described herein can likewise be assigned to other claimed objects and vice versa. In other words, claims addressing the inventive method can be improved by features described or claimed with respect to the apparatuses. In this case, e.g., functional features of the method are embodied by objective units, modules, or elements of the apparatus.

The technical solution will be described both with regard to methods and systems for identifying medical findings in medical data sets and also with regard to methods and systems for providing trained functions. Features and alternate forms of embodiments of data structures and/or functions for methods and systems for identification can be transferred here to analogous data structures and/or functions for methods and systems for providing trained functions. Analogous data structures can in particular be identified here by using the prefix "training". Furthermore, the trained functions used in methods and system for identifying one or more slices in a medical image data set can in particular have been adjusted and/or trained and/or provided by methods and systems for adjustment of trained functions.

According to an aspect, a method for providing an updated machine learning algorithm comprising the following steps is provided:
- providing a medical data set comprising at least one medical finding;
- retrieving, from a database, a medical report associated with the medical data set, the medical report comprising at least one indication of the at least one medical finding;
- obtaining a natural language processing algorithm configured to extract indications of medical findings from structured and/or unstructured text;
- applying the natural language processing algorithm to the medical report so as to extract the at least one indication of the at least one medical finding;
- obtaining a machine learning algorithm configured to extract medical findings from medical data sets;
- updating the machine learning algorithm based on the medical data set and the extracted at least one indication; and
- providing the updated machine learning algorithm,
wherein the step of updating (S60) comprises:
- applying (S61) the machine learning algorithm to the medical data set so as to extract a medical finding from the medical data set corresponding to the at least one medical finding;
- comparing (S62) the extracted medical finding to the at least one indication of the at least one medical finding;
- updating the machine learning algorithm based on the step of comparing
- determining (S63) a correspondence level between the extracted medical finding and the at least one indication; wherein
- the machine learning algorithm is only updated if the correspondence level is above a predetermined threshold.

Machine learning algorithms, in general, may be seen as mapping input data to output data thereby fulfilling a certain learned task. According to some examples, the machine learning algorithm may be configured to carry out one or more of the following tasks: respectively extract one or more data descriptors from medical data sets and classify one or more data descriptors. The relation between input and output may be governed by one or more (in general: a plethora) of parameters embedded in the machine learning algorithms. The values of the parameters may be learned (adapted) during training according to the task, the machine learning algorithms will have to fulfill. Other terms for machine learning algorithms may be trained mapping specification, mapping specification with trained parameters, function with trained parameters, trained machine learned model, algorithm based on artificial intelligence, or trained function.

According to some examples, the trained function comprises a machine learned and/or learnable (artificial) neural network, most preferably a convolutional neural network. A neural network is basically built up like a biological neural net, e.g., a human brain. In particular, an artificial neural network comprises an input layer and an output layer. It may further comprise a plurality of layers between input and output layer. Each layer comprises at least one, preferably a plurality of nodes. Each node may be understood as a biological processing unit, e.g., a neuron. In other words, each neuron corresponds to an operation applied to input data. Nodes of one layer may be interconnected by edges or connections to nodes of other layers, in particular, by directed edges or connections. These edges or connections define the data flow between the nodes of the network. In particular, the edges or connections are equipped with a parameter, wherein the parameter is often denoted as "weight". This parameter can regulate the importance of the output of a first node to the input of a second node, wherein the first node and the second node are connected by an edge. In particular, a neural network can be trained. In particular, training of a neural network is performed based on known pairs of input and output values according to a 'supervised learning' technique, wherein the known input values are used as inputs of the neural network, and wherein the corresponding output value of the neural network is compared to the corresponding known output value. The artificial neural network independently learns and adapts the weights for the individual nodes as long as the output values of the last network layer sufficiently correspond to the known output values according to the trainings data. For convolutional neural networks, this technique is also called 'deep learning'. The terms 'neural network' and 'artificial neural network' can be used as synonyms.

A first group of neural network layers may be applied to extract features from data items comprised in the medical data sets, in particular, from respective image data, text data and/or longitudinal data. Image data may, for instance, be given in the form of the gray scale and/or color values of each slice/image. The thus extracted features like, contrast, gradients, texture, density, distortion, singularities, patterns, landmarks, masks or the like may form an image descriptor of the respective image/slice. The image descriptors may be fed as input values to a second group of network layers which serve to determine a degree of similarity between two slices or a slice and a key image based on the extracted features. However, both functions of the described neural network may likewise be carried out by separated, individual neural networks. In other words, image analysis for feature extraction can be carried out by a first neural network, and classification according to similarity, i.e., object and/or characteristic assignment, can be carried out by a second neural network.

The machine learning algorithm may be a medical findings detection algorithms generally be configured to detect candidate medical findings in medical images. For instance, the findings detection algorithms may have two stages: the detection stage for detecting potentially relevant patterns in image data and the classification stage one for classifying the potentially relevant patterns either as candidate medical findings or as false positives to be discarded. In principle, a plethora of functionalities and methods is known for such computer aided detection and classification of candidate medical findings - all of which may be implemented in the findings detection algorithms. For instance, reference is made to US 2009 / 0 092 300 A1, US 2009 / 0 067 693 A1, and US 2016 / 0 321 427 A1. In particular, findings detection algorithms may comprise one or more machine learned functions trained to detect and classify candidate medical findings if applied to a medical image.

Suitable trained functions for this task include (artificial) neural networks, such as convolutional neural networks.

In general, medical data sets comprise any information which could be helpful in the underlying task of coming to an appropriate clinical decision. As such, the medical data sets may comprise personal details of the target patient (such as age, gender, habits, insurance details, etc.). Further, the medical data sets may comprise the health record of the target patient, e.g., in the form of an electronic medical record (EMR). Moreover, the medical data sets may comprise data which has been measured in one or more medical examinations, which may comprise non-image data and image data. The non-image data may be lab data, such as gene sequences obtained from a biopsy sample, blood values, cardiovascular values and so forth. The image data may be radiology image data. The radiology image data may relate to two-dimensional image data providing two dimensions in space. Further, the radiology image data may relate to three-dimensional image data providing three dimensions in space. In general, the radiology image data depicts a body part of a patient in the sense that it contains two- or three-dimensional image data of the patient's body part. The radiology image data may, for example, be in the form of an array of pixels or voxels. Such arrays of pixels or voxels may be representative of intensity, absorption or other parameter as a function of three-dimensional position, and may, for example, be obtained by suitable processing of measurement signals obtained by a medical imaging modality. A medical imaging modality corresponds to a system used to generate or produce medical images. For example, a medical imaging modality may be a computed tomography system (CT system), a magnetic resonance system (MR system), an angiography (or C-arm X-ray) system, a positron-emission tomography system (PET system) or the like. Besides radiology image data, the patient data may comprise digital pathology image data such as histopathology images acquired with a digital pathology slide scanner. Histopathology images may relate to slices from biopsy samples of the target patient which have been stained with one or more pigments. The most commonly used stain in histopathology is a combination of hematoxylin and eosin (often abbreviated H&E). Other compounds used to color tissue sections include safranin, Oil Red O, congo red, silver salts and artificial dyes. Recently, also antibodies are used for specifically identifying categories of cells in digital pathology images.

The medical data sets may be received from a healthcare information system comprising one or more databases and servers. The healthcare information system may be a local system within the premises of a healthcare organization. Moreover, the healthcare information system may be cloud based. The healthcare informatics system may comprise one or more standardized components such as a picture archiving and communication system (PACS), a radiology information system (RIS), a laboratory information system (LIS), a hospital information system (HIS), or an electronic medical record system (EMR). Image data comprised in the patient data may be formatted according to the DICOM format. DICOM (=Digital Imaging and Communications in Medicine) is an open standard for the communication and management of medical imaging information and related data in healthcare informatics. DICOM may be used for storing and transmitting medical images and associated information enabling the integration of medical imaging devices such as scanners, servers, workstations, printers, network hardware, and picture archiving and communication systems (PACS). It is widely adopted by clinical syndicates, hospitals, as well as for smaller applications like doctors' offices or practices. A DICOM data object consists of a number of attributes, including items such as patient's name, ID, etc., and also special attributes containing the image pixel data and metadata extracted from the image data. For describing data formats and exchanging electronic health records, corresponding standards may be relied upon such as the HL7 FHIR standard. Further, besides designated databases, the healthcare informatics system may directly involve medical imaging modalities such as radiology and/or pathology imaging systems.

A medical report may comprise structured or unstructured text in natural language. A medical report may summarize the findings for a patient with one or more words. Each medical report may relate to a designated patient and/or medical data set.

"Retrieving" in the framework of the application may mean that the medical report(s) is acquired from the healthcare information system. Thereby, the resources of the healthcare information system may be queried separately, e.g., on the basis of the target patient's ID or other suitable identifier. Moreover, a concerted query may be sent to the healthcare information system which may be configured to manage such data requests internally.

Each medical finding may relate to corresponding data item in the medical data set. A medical finding may indicate a certain condition or pathology of the patient. The condition or pathology may be relevant for the diagnosis of the patient. In addition, the medical finding may indicate certain workflow steps to be carried out in diagnosis and/or treatment of the patient.

A medical finding may relate to an anatomical structure that differentiates the patient from other patients. Medical findings may be located within different organs of the patient (e.g., within the lung of a patient, or within the liver of a patient) or in between the organs of the patient. In particular, a medical finding may also relate to a foreign body.

In particular, a medical finding may relate to a neoplasm (also denoted as "tumor"), in particular, a benign neoplasm, an in-situ neoplasm, a malignant neoplasm and/or a neoplasm of uncertain/unknown behavior. In particular, a medical finding may relate to a nodule, in particular, a lung nodule. In particular, a medical finding may relate to a lesion, in particular, a lung lesion.

A indication of a medical finding may relate to any piece of information comprised in the medical report comprising information of a medical finding such as type, characteristics and/or locations of the medical findings in the medical data set. In particular, an indication may be comprised in the medical report in the form of one or more words or sentences.

The natural language processing algorithm may generally be configured to process natural language and output results which are machine readable such as in the form of the indications.

A transformer network is a neural network architecture generally comprising an encoder, a decoder or both an encoder and decoder. In some instances, the encoders and/or decoders are composed of several corresponding encoding layers and decoding layers, respectively. Within each encoding and decoding layer is an attention mechanism. The attention mechanism, sometimes called self-attention, relates data items (such as words or pixels) within a series of data items to other data items within the series. The self-attention mechanism for instance allows the model to examine a group of words within a sentence and determine the relative importance other groups of words within that sentence have to the word being examined. For a review on transformer networks, reference is made to Vaswani et al., "Attention Is All You Need", in arXiv: 1706.03762, June 12, 2017.

Updating the machine learning algorithms may mean adjusting one or more parameters of the machine learning algorithm by machine learning. In particular, updating may comprise further training the machine learning algorithm.

With the above method, the medical data available in an organization may automatically be mined for training data sets for further training the machine learning algorithm. With that, the requirement for manual annotations may be reduced and the further training of machine learning algorithms may be rendered more efficiently.

It is an idea of the invention to use natural language processing techniques to improve the training in of machine learning algorithms in the medical environment. Natural language processing (NLP) techniques have the potential to automatically analyze medical reports, including radiology reports. One approach to achieve this is through the use of large language models. Such language models have been shown to be effective at tasks such as text generation and language translation. It has also been applied to the analysis of medical texts, including the extraction of information from electronic medical records as exemplified in Miotto, R. et al., "Deep patient: An unsupervised representation to predict the future of patients from the electronic health records", in Scientific Reports, volume 6, Article number: 26094 (2016)

To use natural language processing algorithms for the analysis of radiology reports, the text of the report would be input into the algorithm. The natural language processing algorithms would then be trained to identify mentions of diseases and other relevant information, such as anatomic location. This information would be output as structured data for further analysis and interpretation in combination with knowledge about the position of anatomic location expressions, e.g., according to a body atlas. With further integration of algorithms, e.g., an ontology service on the disease mentioned, relevant findings for the subsequent step can be selected.

The algorithms used are furthermore combined to deliver a probability towards the accuracy of the identified diseases or patterns and the precision on the anatomic location. This may comprise textual interpretation but also the mentioning of concrete positioning information in the report (slice 234 in series 4) as well as the relation to anatomic landmarks (3cm distance to ...).

This information is then used to retrain the machine learning algorithm which may comprise disease identification and classifying machine learning networks (CAD). In an embodiment this can done as so-called federated learning. The probability information from the natural language processing algorithm and the probability scores of the machine learning algorithm can be used to train and/or validate the machine learning algorithm. In a further embodiment, the training can happen on existing patient data where further outcomes are known and not yet disclosed to the machine learning algorithm to create additional ground truth for training.

According to another aspect, natural language processing algorithms may be combined with anatomic knowhow algorithms, and/or disease knowledge algorithms to train a machine learning algorithm either in a supervised or non-supervised manner.

Further, the step of updating comprises:
- applying the machine learning algorithm to the medical data set so as to extract an intermediate medical finding from the medical data set corresponding to the at least one medical finding;
- comparing the intermediate medical finding to the at least one indication of the at least one medical finding; and
- updating the machine learning algorithm based on the step of comparing.

In other words, the updating can comprise a direct comparison on the basis of the extracted indications. With that, an efficient training can be effected.

Further, the step of updating further comprises determining a correspondence level between the intermediate medical finding and the at least one indication; and the machine learning algorithm is only updated if the correspondence level is above a predetermined threshold.

The correspondence level may be conceived as a measure how well the indication is suited for further training the machine learning algorithm. If there is a huge difference between the intermediate medical finding and the indication, the indication likely pertains to a different medical finding and should not be used for further training the machine learning algorithm. Accordingly, a quality gate is established ensuring that the training is only conducted based on high quality data.

According to an aspect,
- the medical data set comprises a medical image data set;
- the medical finding relates to an image feature, in particular a lesion, depicted in the medical image data set;
- the indication comprises a type and/or location of the image feature in the medical image data set; and
- the machine learning algorithm is configured to extract image features from medical image data sets as medical findings.

In other words, the method may be employed for medical image data where it is particularly useful. To identify the location of the image feature, the natural language processing algorithm may be configured to output an organ in which the medical finding resides.

According to some aspects, the method may further comprise segmenting the medical image data set according to the location and/or organ. That followed the segmented medical image data set may be input into the machine learning algorithm. With that a more targeted training of the machine learning algorithm is possible.

According to other aspects, the step of obtaining a machine learning algorithm may comprise providing a plurality of different machine learning algorithms and selecting one of the different machine learning algorithms based on the indication and, in particular, the location. For instance, the plurality of machine learning algorithms may differ in that they have been trained to detect medical findings in different organs, respectively. Accordingly, the selection of the machine learning algorithm may involve selecting a machine learning algorithm from the plurality of machine learning algorithms which have been configured to detect medical findings in the organ comprised in the indication.

Of course, the above concept of segmentation may also be applied to non-image data.

Method according to any one of the preceding claims, wherein
- the indication comprises a probability value for the presence of the medical finding in a data item of the medical data set.

The inventors have realized that providing probability values enables a comparison of data structures which are difficult to compare such as indications taken form text data and medical findings extracted from image and/or non-image data.

The data item may, in particular be a pointer to a given location in the corresponding medical data set, such a study and slice number and/or a distance to one or more anatomic landmarks.

According to an aspect,
- the machine learning algorithm is configured to output a probability map for the presence of medical findings in different data items of the medical data set so as to extract medical findings from the medical data set, and
- the step of further training is based on a comparison of the probability value and the probability map.

By matching the probability value with the probability map, the outputs of the natural langue processing algorithm and the machine learning algorithm can be readily compared.

According to an aspect,
- the natural language processing algorithm is based on a transformer model; and/or
the machine learning algorithm is based on a convolutional neural network.

An advantage of transformer networks is that, due to the attention mechanism, transformer networks can efficiently deal with long-range dependencies in input data. Further, encoders used in transformer networks are capable of processing data in parallel which saves computing resources in inference. Moreover, decoders of transformer networks, due the auto-regression, are able to iteratively generate a sequence of output tokens with great confidence.

By using convolutional neural networks, input images can be processed in a very efficient way, because a convolution operation based on different kernels can extract various image features, so that by adapting the weights of the convolution kernel the relevant image features can be found during training. Furthermore, based on the weight-sharing in the convolutional kernels less parameters need to be trained, which prevents overfitting in the training phase and allows to have faster training or more layers in the network, improving the performance of the network.

According to an aspect, a method for providing an updated machine learned algorithm form a client computing device to a model aggregator device is provided, the method comprising the following steps:
- obtaining, at the client computing device from the model aggregator device, a machine learned algorithm, the machine learned algorithm being configured to extract medical findings from medical data sets;
- obtaining, at the client computing device from the model aggregator device a natural language processing algorithm, the natural language processing algorithm being configured to extract indications of medical findings from text data;
- obtaining, at the client computing device, a set of medical data sets each comprising at least one medical finding;
- retrieving, by the client device from a database, a set of medical reports respectively associated with a medical data set of the set of medical data sets;
- applying, by the client computing device, the natural language processing algorithm to the set of medical reports so as to extract indications of the medical findings from the medical reports, each indication corresponding to a medical data set;
- determining, by the client computing device, whether to allow a local data item comprising one of the medical data sets and a corresponding indication to be incorporated into a training process of the machine learning algorithm;
- updating the machine learning algorithm using the allowed local data items;
- providing the updated machine learning algorithm from the local client device to the model aggregator device.

According to an aspect, a method for providing an updated machine learned algorithm form a client computing device to a model aggregator device is provided, the method comprising the following steps:
- obtaining, at the client computing device from the model aggregator device, a machine learned algorithm, the machine learned algorithm being configured to extract medical findings from medical data sets;
- obtaining, at the client computing device from the model aggregator device a natural language processing algorithm, the natural language processing algorithm being configured to extract indications of medical findings from text data;
- obtaining, at the client computing device, a set of medical data sets each comprising at least one medical finding;
- retrieving, by the client device from a database, a set of medical reports respectively associated with a medical data set of the set of medical data sets;
- applying, by the client computing device, the natural language processing algorithm to the set of medical reports so as to extract indications of the medical findings from the medical reports, each indication corresponding to a medical data set;
- updating the machine learning algorithm based on the medical data set and the extracted at least one indication; and
- providing the updated machine learning algorithm from the local client device to the model aggregator device.

The model aggregator device may, in particular, relate to a web server. Further, the model aggregator device may be a cloud server or a local server. The client devices may, in particular, relate to a local computer network at the local sites comprising one or more computing units. The local sites may, for instance, relate to healthcare environments or facilities such as hospitals, laboratories, practices, universities or associations of one or more of the aforesaid. In general, the model aggregator device is located outside of the local sites and serves one or more of the local sites / client devices from the outside.

The above claim brings together the automated generation and quality control of training data and the concept of federated learning. With that, the two concepts synergistically contribute to a more effective update of machine learning algorithm. In particular, the model aggregator device may be configured to integrate the updated machine learning algorithm into a master algorithm with basically the same functionality of the updated machine learning algorithm. Decentralized training alleviates data privacy and regulatory concerns associated with the collation of data to a centralized server. It also addresses the factors that contribute to the heterogeneity of data such as geographical origin, annotation practices, experience of the expert and the like.

Specifically, a central machine learned algorithm (or master model) hosted at the (central) model aggregator device may be improved based on the usage reported by many client devices. Thereby, a readily trained, functioning, and deployable master model is distributed to the client device and executed locally. Each client device randomly, periodically, or on command may send a local update to the model aggregator device. The local update may summarize a local change to the machine learned algorithm based on the local data gathered by the client device. The model aggregator device may use the local updates to improve the machine learned model. In turn, the model aggregator device may then download a modified machine learned algorithm that implements the learning modification based on actual usage reported by the client devices. This enables the client devices to collaboratively learn and improve a shared machine learned model. Thereby different client device may belong to different medical organizations such as hospitals, practices, or chains of the aforesaid.

According to an aspect, the step of determining comprises:
- applying the machine learning algorithm to the medical data sets so as to extract, from each medical data set, at least one medical finding, and
- the step of determining whether to allow is additionally based on the extracted medical findings and, in particular, comprises comparing the extracted medical findings with the extracted indications.

By basing the training data selection on the extracted medical findings, wrong indications can be filtered. Therewith it can be ensured that the local training data is not contaminated.

According to an aspect, the step of determining comprises applying a quality gating algorithm to the extracted indications, and
- the quality gating algorithm is obtained by the client computing device from the model aggregator device.

With that, means for filtering the data items can be centrally provided thereby ensuring a high data integrity.

According to an aspect, the client computing device is configured as an Edge device in a medical information system comprising the database.

With that, the data of the medical information system can efficiently be used.

According to an aspect, the model aggregator device is a server device remote from the client computing device.

According to an aspect, the model aggregator device is configured to modify a master model of the machine learning algorithm based on the updated machine learning algorithm.

A local model update module is provided comprising:
- an interface configured to:
   obtain, from a model aggregator device, a machine learned algorithm, the machine learned algorithm being configured to extract medical findings from medical data sets;
   obtain, from the model aggregator device, a natural language processing algorithm, the natural language processing algorithm being configured to extract indications of medical findings from text data;
   obtain, form one or more local databases, medical data sets and associated medical reports;
- a data parsing module configured to query the one or more local databases for a set of medical data sets and a set of associated medical reports, the medical data sets each comprising at least one medical finding;
- a reference data generation module configured to generate indications of at least a part of the medical findings by applying the natural language processing algorithm to the medical reports;
- a data gating module configured to determine whether to allow a local data item comprising one of the medical data sets and a corresponding indication to be incorporated into a training process of the machine learning algorithm;
- a training module configured to update the machine learned algorithm based on the al

### Brief description of the drawings

The interface may comprise an interface for data exchange with a local server or a central web server via internet connection for receiving the medial image data sets. The interface unit may be further adapted to interface with one or more users of the system, e.g., by displaying the result of the processing by the computing unit to the user (e.g., in a graphical user interface) or by allowing the user to adjust parameters for image processing or visualization.

The modules as described before may be comprised in a computing unit. The computing unit may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories and combinations thereof. The one or more memories may store instructions for carrying out the method steps according to the invention. The hardware can be configurable by the software and/or be operable by the software. Generally, all units, sub-units, or modules may at least temporarily be in data exchange with each other, e.g., via a network connection or respective interfaces. Consequently, individual units may be located apart from each other.

According to another aspect, the present invention is directed to a computer program product comprising program elements which induce a computing unit of a system to perform the steps according to one or more of the above method aspects, when the program elements are loaded into a memory of the computing unit.

According to another aspect, the present invention is directed to a computer-readable medium on which program elements are stored that are readable and executable by a computing unit of a system according to one or more method aspects, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

### Brief description of the drawings

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:
FIG. 1 schematically depicts a method for providing an updated machine learning algorithm according to an embodiment,
FIG. 2 schematically depicts a method for providing an updated machine learning algorithm according to an embodiment,
FIG. 3 schematically depicts a system for providing an updated machine learning algorithm according to an embodiment,
FIG. 4 schematically depicts a data flow diagram for providing an updated machine learning algorithm according to an embodiment, and
FIG. 5 schematically depicts a system for providing an updated machine learning algorithm according to an embodiment.

### Detailed description of preferred embodiments

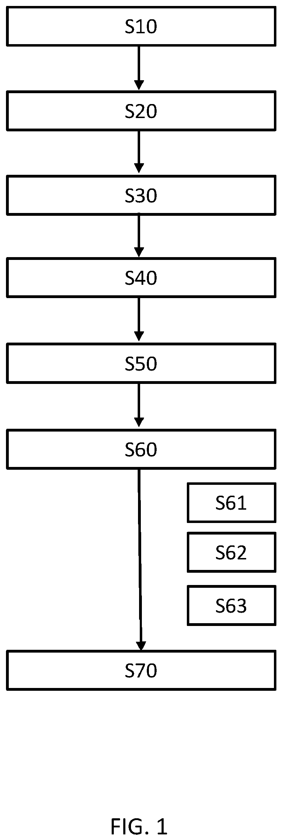

### Figure 1 - Method 1

Figure 1 depicts a method for providing an updated machine learning algorithm U-MLA according to an embodiment.

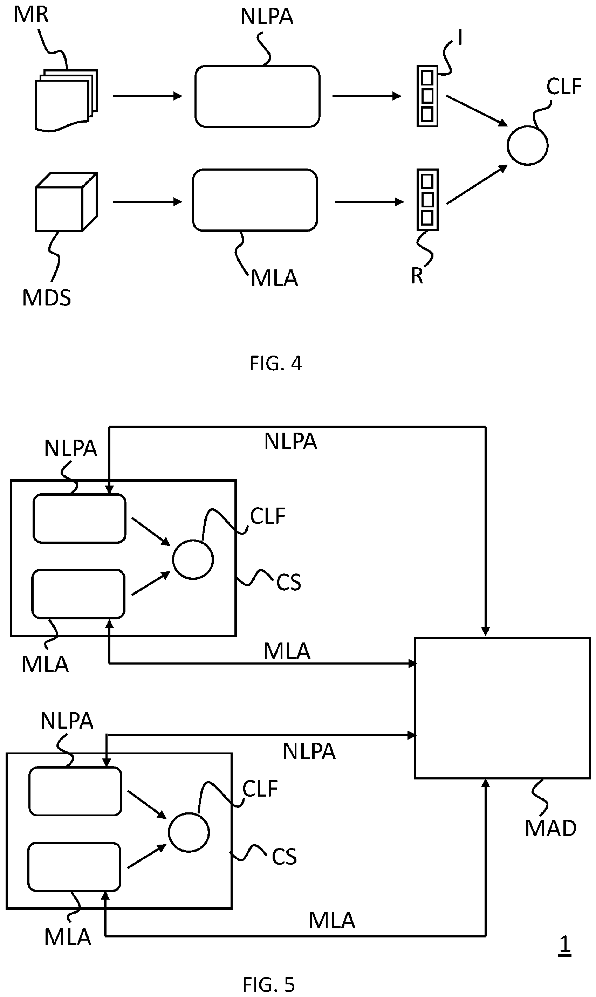

Corresponding data streams are illustrated in Figure 4. The method comprises several steps. The order of the steps does not necessarily correspond to the numbering of the steps but may also vary between different embodiments of the present invention. Further, individual steps or a sequence of steps may be repeated.

At a first step S10, a medical data set comprising at least one medical finding is provided. In particular, at step S10, the medical data set MDS may be retrieved from a database DB2 configured to store one or more medical data sets MDS. At step S20, a medical report RP is retrieved from a report database DB1. Thereby, the medical report MR is associated with the medical data set MDS. "Associated" may mean that the medical data set MDS and the medical report MR have the same patient identifier, i.e., belong to the same patient. Accordingly, step S20 may comprise extracting a patient identifier from the medical data set MDS and using the patient identifier for querying the report database DB1 for the associated medical report MR. The medical report MR may comprise at least one data item describing in writing one or more medical findings comprised in the medical data set MDS.

At step S30, a natural language processing algorithm NLPA is obtained. In particular, the natural language processing algorithm NLPA may be obtained from a model aggregator device MAD at the client computing device CCD. In particular, the natural language processing algorithm NLPA may be configured to process any natural language text comprised in the medical report MR so as to extract one or more indications I therefrom, the indications I, in particular, comprising any one of a type, location, and/or characteristics of the medical finding. In particular, the indications I may encode these properties in a machine-readable format such as in the form of a feature vector (also called embeddings of the medical findings in the medical report MR).

At step S40, the natural language processing algorithm NLPA is applied to the medical report MR so as to extract the at least one indication I of the at least one medical finding.

That followed, the machine learning algorithm MLA is obtained at step S50. In particular, the machine learning algorithm MLA may be obtained from a model aggregator device MAD at the client computing device CCD. The machine learning algorithm MLA may be configured to detect one or medical findings in medical image data sets. The machine learning algorithm MLA may be provided in a readily trained format by the model aggregator device MAD in the sense that it is ready for deployment.

At step S60, the machine learning algorithm MLA is updated based on the medical data set MDS and the extracted at least one indication I and the thus updated machine learning U-MLA algorithm MLA may be provided at step S70. In particular, the updated machine learning algorithm U-MLA may be provided from the client computing device CCD to the model aggregator device MAD at. At the model aggregator device MAD, the updated machine learning algorithm U-MLA may be included in a master model of the machine learning algorithm MLA. That followed, the master model of the machine learning algorithm MLA may be re-distributed to client computing devices CCD as machine learning algorithm MLA.

Step S60 may comprise several optional steps. At step S61, the machine learning algorithm MLA is applied to the medical data set MDS so as to extract an intermediate medical finding from the medical data set MDS which corresponds to the at least one medical finding for which the indications I were obtained. At step S62, the intermediate medical finding is compared to the indication I. Since the comparison gives insights who good or bad the machine learning algorithm is performing, the comparison can, in principle, be used to update the machine learning mode. Self-speaking, the steps S61 and S62 are repeated with paired sets of indications I and medical data sets MDS until the machine learning algorithm MLA is able to generate results that are acceptable (i.e., until a local minimum of a loss function is reached). Once all pairs have been used, pairs are randomly shuffled for the next pass.

As a safety check, a correspondence test may be performed at step S63 based on a comparison of the indication I and the intermediate medical finding to check if the two are at least related in the sense that they are located at roughly the same locations. If no match between the indication I and the intermediate medical finding can be found, the data item can be discarded. In particular, the indication I may comprise a probability or confidence value for the presence of the medical finding in a data item of the medical data set MDS and the machine learning algorithm MLA is configured to output a probability or confidence value for the presence of medical findings in data items of the medical data set MDS. Then, step S63 may comprise allowing only those data pairs into the training data, the confidence values of which is independently is above predetermined thresholds.

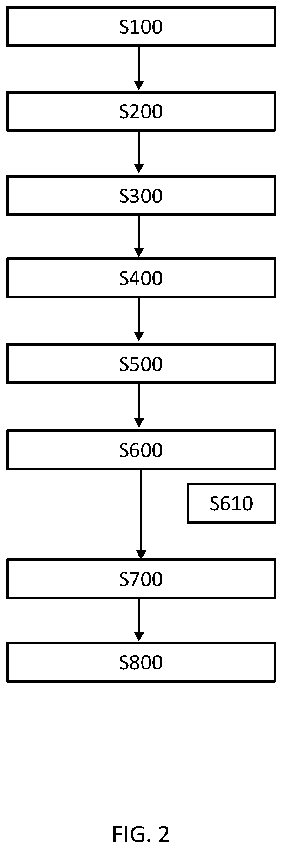

### Figure 2 - Method 2

Figure 2 depicts a method for providing an updated machine learning algorithm U-MLA form a client computing device CCD to a model aggregator device MAD according to an embodiment. Corresponding data streams are illustrated in Figure 4. The method comprises several steps. The order of the steps does not necessarily correspond to the numbering of the steps but may also vary between different embodiments of the present invention. Further, individual steps or a sequence of steps may be repeated.

At step S100 a machine learned algorithm MLA is obtained at the client computing device CCD from the model aggregator device MAD. As before, the machine learned algorithm MLA is configured to extract medical findings from medical data sets MDS.

At step S200, a natural language processing algorithm NLPA being configured to extract indications I of medical findings from text data is received at the client computing device CCD from the model aggregator device MAD.

At step S300, a set of medical data sets MDS each comprising at least one medical finding are obtained at the client computing device CCD, for instance, by querying the database DB2.

At step S400, a set of medical reports MR is retrieved by the client computing device CCD, for instance, from the report database DB1. Thereby, the medical reports MR thus retrieved are respectively associated to at least one medical data set MDS in the sense that each medical report MR can unambiguously be assigned to a medical data set MDS.

That followed, at step S400, the natural language processing algorithm NLPA is applied to the set of medical reports MR by the client computing device CCD so as to extract indications I of the medical findings from the medical reports MR. Like each report corresponds to a medical data set, each indication I corresponds to a medical data set MDS in the sense that it can unambiguously be assigned to a medical data set MDS.

At step S600, it is determined, by the client computing device CCD, whether to allow a local data item or data pair comprising one of the medical data sets MDS and a corresponding indication I to be incorporated into a training process of the machine learning algorithm MLA. This may mean that the data pair may be adopted in the local training data of the client computing device CCD. Optionally, this may comprise at step S610 applying the machine learning algorithm MLA to the medical data sets MDS so as to extract, from each medical data set MDS, at least one (intermediate) medical finding. These intermediate medical findings may then be used to determining whether to allow a data pair to the training process, for instance, based on corresponding confidence or probability values or on a comparison of the extracted medical findings with the extracted indications. According to some examples, step S610 may be performed by applying a gating algorithm to the extracted indications I and/or medical data sets MDS and/or intermediate medical findings which may likewise be provided as yet another tool from the model aggregator device MAD to the client computing device CCD.

That followed, at step S700, the machine learning algorithm MLA may be updated using the local training data, i.e., the data items or pairs allowed for the training process. For instance, updating a machine learning algorithm MLA may comprise (further) training the algorithm to generate a further trained machine learning algorithm and evaluating how it performs on the local data. This may involve generating a plurality of candidate machine learning algorithm MLA and comparing them on the basis of the local (training) data to decide about the best final updated machine learning algorithm U-MLA. To this end, cross-validation schemes may be used according to which the local training data is partitioned into a plurality of complementary subsets or folds. Training is performed on one subset (called the training set or training fold) and testing is performed on the other subset (called the testing set or fold). To reduce variability, multiple rounds of cross-validation may be performed using different partitions of the local training data, and the validation results are combined (e.g., averaged) over the different partitions to give an estimate of the machine learned model's predictive performance. More details as to how a local update of the machine learning algorithm MLA may be performed can be found in EP 3 798 934 A1.

Finally, at step S800, the updated machine learning algorithm MLA is provided from the local client computing device CCD to the model aggregator device MAD.

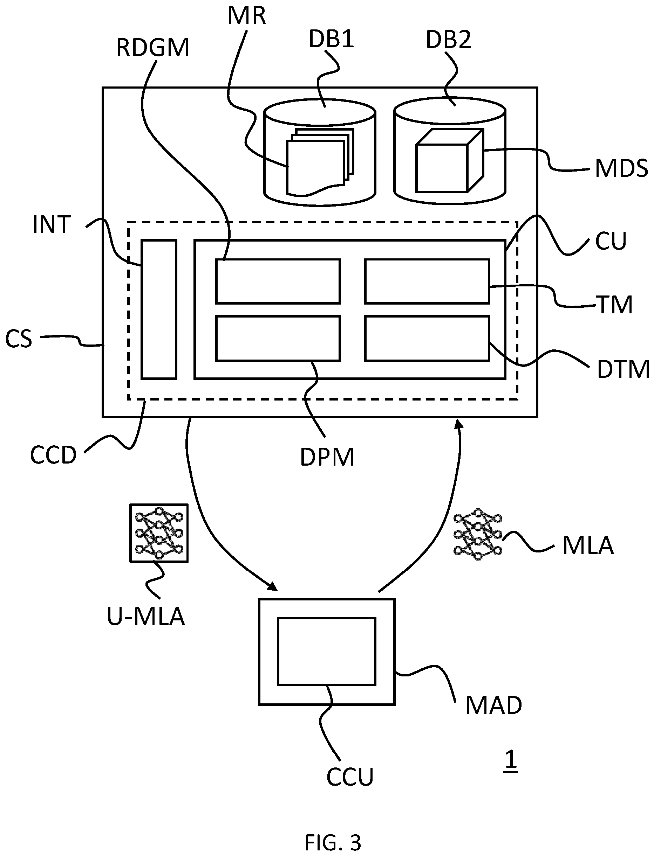

### Figure 3 - System

Figure 3 depicts an example system 1 for client-specific federated learning capable of training, updating, distributing, monitoring and, generally, administrating a plurality machine learning algorithms MLA in an environment comprising one or more client systems CS (for the sake of easy reference only one is shown) at (different) local sites. System 1 is adapted to perform the method according to one or more embodiments, e.g., as further described with reference to Figures 1, 2, 4 and/or 5.

System 1 comprises a model aggregator device MAD and at least one client system CS located at a local site. Aggregator device MAD and client system CS are interfaced via a network. The model aggregator device MAD may generally configured to control, coordinate and steer the federated learning procedures in system 1. The federated learning procedures may comprise creating, training, updating, distributing, deploying, monitoring and, generally, administrating machine learning algorithms MLA at the client system CS. The local site may, for instance, relate to clinical or medical environments, such as hospitals or hospital groups, laboratories, medical image centers, clinics or practices.

The machine learning algorithms MLA may be seen as mimicking cognitive functions that humans associate with other human minds. In system 1, such functions may relate to medical diagnosis, outcome prediction, the generation of findings in medical image data and non-image data, the annotation of medical images, e.g., in terms of orientations or landmark detection, the generation of medical reports, recommendations for next steps in a clinical workflow and the like. The machine learning algorithms MLA is enabled to fulfill these functions by being generated from one or more basic trainable machine learning algorithms and being trained based on local data I, MDS at the client system CS. The trainable machine learning algorithms stored in algorithm repository 122 may include modules predominately configured for classifying data and/or for generating numerical predictions and/or clustering data. Specifically, the algorithms may include (or may be based on) one or more neural networks (e.g., deep neural networks, recurrent neural networks, convolutional neural networks, convolutional deep neural networks, adversarial networks, deep adversarial networks and/or a generative adversarial networks etc.) and/or other algorithms including Bayesian networks, decision trees, random forest schemes, linear or logistic regression models, gradient averaging, k-means clustering, Qlearning, genetic algorithms and/or association rules or other suitable models.

Model aggregator device MAD may be a web server, for instance. Further, the model aggregator device MAD may be a cloud server or a local server. Model aggregator device MAD may be implemented using any suitable computing device(s). Model aggregator device MAD may have a computing unit CCU which is configured to provide the machine learning algorithm MLA, the natural language processing algorithm NLPA and other tools to the client system CS. Further, the computing unit CCU of the model aggregator device MAD may be configured to incorporate an updated machine learning algorithm MLA in a master model.

The computing unit CCU of the model aggregator device MAD may comprise one or more processors and a working storage. The one or more processor(s) may include, for example, one or more central processing units (CPUs), graphics processing units (GPUs), and/or other processing devices. Computing unit CCU may further comprise a micro-controller or an integrated circuit. Alternatively, computing unit 110 may comprise a real or virtual group of computers like a so called 'cluster' or 'cloud'. Model aggregator device MAD may further include an interface unit (not shown) for communication with the client system CS over the network. The interface unit can include any suitable components for interfacing with one or more networks, including, for example, transmitters, receivers, ports, controllers, antennas, or other suitable components.

The client system CS comprises a client computing device CCD two databases DB1 and DB2 configured as the local memory unit of the client system CS. The databases DB1, DB2 may be realized as a cloud storage accessible via the corresponding client computing device CCD. Alternatively, the databases DB1, DB2 may be realized as a local or spread storage within the premises of the respective client system CS. Further databases DB1 and DB2 may also be integrated in one database or memory unit.

The database DB2 is configured to store medical data sets MDS.

The medical data sets MDS may comprise a number of data sets relating, for instance, to a clinical or medical problem. As an example, the data sets MDS may relate to laboratory test results and/or pathological data and/or medical imaging data and any combination thereof. The medical data sets MDS may relate to medical data of one or more patients. For instance, the medical data sets MDS may comprise laboratory test results and/or pathological data stemming from pathological imaging and/or medical imaging data generated by one or more medical imaging facilities such as computed tomography devices, a magnetic resonance system, an angiography (or C-arm X-ray) system, a positron-emission tomography system or the like and any combination thereof. The medical data sets MDS may be generated at the respective local site of the client system CS. Further, the medical data sets MDS may comprise non-image data relating to a patient, information about administered treatments, information about symptoms and treatment responses, health progression and the like. Such information can be provided by ways of an electronic medical record (EMR), for instance. The medical data sets MDS may be stored locally in one or more databases of the client system CS. The databases may be part of a hospital information systems (HIS), radiology information systems (RIS), clinical information systems (CIS), laboratory information systems (LIS) and/or cardiovascular information systems (CVIS), picture archiving and communicating system (PACS) or the like. From these databases, the medical data sets MDS can be accessed locally for training machine learned models and the later regular use of the machine learned algorithm MLA after deployment. Further, the client system CS comprises a report database DB1 configured to store one or more medical reports MR. Thereby, the medical reports MR contain observations made or at least verified by humans in a human-readable form such as in the form of one or more structured or unstructured text blocks. Of note, the medical reports may be stored alongside the medical data sets MDS in one or more databases of the client system CS as described above and not necessarily in a separate database. The databases DB1, DB2 may be updated continuously or on a daily or weekly basis, or, in general, whenever new test results become available.

The local access of the medical data sets MDS and the medical reports MR and, in particular, their delivery to the machine learning algorithm MLA and natural language processing algorithm NLPA may be administered or controlled by the client computing unit CU. The client computing unit CU can be any suitable type of computing device, such as a general-purpose computer, special purpose computer, laptop, local server system, or other suitable computing device. Client computing unit CU may include one or more processor(s) and a memory. The one or more processor(s) may include, for example, one or more central processing units (CPUs), graphics processing units (GPUs), and/or other processing devices. The memory can include one or more computer-readable media and can store information accessible by the one or more processors, including instructions that can be executed by the one or more processors. The instructions can include instructions for fetching medical data sets MDS and/or medical reports MR, the application of the machine learning algorithm MLA or the natural language processing algorithm NLPA, and/or updating the machine learning algorithm MLA.

Further, the client computing device CCD includes an interface INT. The interface INT may be a network interface used to communicate with the model aggregator device MDA over a suitable network and/or any local databases DB1, DB2. The interface INT can include any suitable component for interfacing with one more network, including for example, transmitters, receivers, ports, controllers, antennas, or other suitable components.

The client computing unit CU may comprise modules configured to carry out the different tasks in the workflow for proving an updated machine learning algorithm MLA.

For instance, the client computing unit CU may comprise a data parsing module DPM configured to query the one or more local databases for a set of medical data sets MDS and a set of associated medical reports MR.

Further, the client computing unit CU may comprise a reference data generation module RDGM configured to generate indications I of at least a part of the medical findings by applying the natural language processing algorithm NLPA to the medical reports MR.

Further, the client computing unit CU may comprise a data gating module DTM configured to determine whether to allow a data pair comprising one of the medical data sets MDS and a corresponding indication I to be incorporated into a training process of the machine learning algorithm MLA.

Further, the client computing unit CU may comprise a training module TM configured to update the machine learned algorithm based on the allowed data pairs.

The designation of the distinct sub-units DPM, DTM, TM, RDGM is to be construed by ways of example and not as limiting the disclosure. Accordingly, sub-units DPM, DTM, TM, RDGM may be integrated to form one single processing unit or can be embodied by computer code segments configured to execute the corresponding method steps running on a processor or the like of computing unit CU. Each sub-unit DPM, DTM, TM, RDGM may be individually connected to other sub-units and or other components of the system 1 where data exchange is needed to perform the method steps. For example, sub-unit DPM may be connected to the databases DB1, DB2.

The flowcharts of Figures 1 and 2 are likewise representative of example hardware logic or machine-readable instructions for implementing the method steps in the form of one or more computer programs. Programs may be embodied in software stored on a non-transitory computer readable storage medium such as a CD-ROM, a floppy disk, a hard drive, a DVD, a Blu-ray disk, or a memory associated with the processor. The entire program and/or parts thereof could alternatively be executed by a device other than the processor and/or embodied in firmware or dedicated hardware.

### Figure 4 - Data Flow

In Figure 4, a data flow diagram for updating a machine learning algorithm MLA according to an embodiment is shown.

A weakly-supervised method for automatic training of an machine learning algorithm MLA is provided. In particular, the machine learning algorithm MLA may be an abnormality detection algorithm. Specifically, textual information in medical reports MR are used as supervisory clues to guide an abnormality segmentation system which identifies the location and extent of abnormal patterns in the input images. The training of the segmentation system is performed using contrastive learning by maximizing the similarity between the input images and the corresponding text prompts in an embedding space.

To this end, a machine learning algorithm MLA and a natural language processing algorithm NLPA may be used to map the visual and textural information from images and reports to a shared representation space. In particular, the machine learning algorithm MLA may comprise a vision transformer. The natural language algorithm NLPA may be based on the reference by Devlin, J. et al. "Bert: Pre-training of deep bidirectional transformers for language understanding" arXiv:1810.04805. The vision transformer may be based on the reference by Dosovitskiy, A., et al. "An Image is Worth 16x16 Words: Transformers for Image Recognition at Scale" in International Conference on Learning Representations.

During training, the similarity between positive/negative image-text embedding pairs is maximized/minimized by optimizing a contrastive loss function CLF.

According to an embodiment, the machine learning algorithm MLA is a system for training a CAD algorithm for abnormality segmentation tasks. The machine learning algorithm MLA may use a multi-layer perceptron head to acquire embedding representations R of input images during training. Likewise, the natural language processing algorithm NLPA is configured to acquire indications I as embeddings of the text within the medical reports MR. According to some examples, the system can be used to train a separate student segmentation model using knowledge distillation techniques.

### Figure 5 - System 2

Figure 5 depicts an example system 1 for client-specific federated learning according to another embodiment. According to this embodiment, the machine learning algorithm MLA as well as the natural language processing algorithm NLPA are further trained in a decentralized manner.

The proposed approach enables training of CAD systems without manual annotation of input images, providing significant advantage for cost savings, scalability and generalizability of the CAD systems. It allows to train a CAD system on the only currently validated outcome of radiology, which is the written report without the explicit creation of additional information such as human annotations.

The system can also be set up to automatically suppress or remove pairs of image and report/EMR data that have a low correlation (e.g. where the textual description is not precise enough). By that it can automatically focus to relevant data pairs found.

Such a system may also be used to obtain train / improve a workflow optimization or outcome-predictor algorithm by training it retrospective on data / reports obtained over several imaging studies.

## Claims

1. Method for providing an updated machine learning algorithm comprising the following steps:
- providing (S10) a medical data set (MDS) comprising at least one medical finding;
- retrieving (S20), from a database (DB-R), a medical report (MR) associated with the medical data set, the medical report comprising at least one indication of the at least one medical finding;
- obtaining (S30) a natural language processing algorithm (NLPA) configured to extract indications of medical findings from structured and/or unstructured text;
- applying (S40) the natural language processing algorithm to the medical report so as to extract the at least one indication (IND) of the at least one medical finding;
- obtaining (S50) a machine learning algorithm (MLA) configured to extract medical findings (MF) from medical data sets;
- updating (S60) the machine learning algorithm based on the medical data set and the extracted at least one indication; and
- providing (S70) the updated machine learning algorithm,
wherein the step of updating (S60) comprises:
- applying (S61) the machine learning algorithm to the medical data set so as to extract a medical finding from the medical data set corresponding to the at least one medical finding;
- comparing (S62) the extracted medical finding to the at least one indication of the at least one medical finding;
- updating the machine learning algorithm based on the step of comparing,
- determining (S63) a correspondence level between the extracted medical finding and the at least one indication; wherein
- the machine learning algorithm is only updated if the correspondence level is above a predetermined threshold.

2. Method according to claim 1, wherein the correspondence level comprises a measure for the difference between the extracted medical finding and the indication.

3. Method according to any one of the preceding claims, wherein
- the medical data set comprises a medical image data set;
- the medical finding relates to an image feature, in particular a lesion, depicted in the medical image data set;
- the indication comprises a type and/or location of the image feature in the medical image data set; and
- the machine learning algorithm is configured to extract image features from medical image data sets as medical findings.

4. Method according to claim 3, wherein the step of updating comprises:
comparing (S63) the indication and the extracted medical finding to check if the two are at least related in the sense that they are located at roughly the same locations.

5. Method according to any one of the preceding claims, wherein,
the natural language processing algorithm is based on a transformer model; and/or
the machine learning algorithm is based on a convolutional neural network.

6. Local model update module (CCD) comprising:
- an interface (INT) configured to:
obtain, from a model aggregator device (MAD), a machine learned algorithm, the machine learned algorithm being configured to extract medical findings from medical data sets;
obtain, from the model aggregator device, a natural language processing algorithm, the natural language processing algorithm being configured to extract indications of medical findings from text data;
obtain, form one or more local databases, medical data sets and associated medical reports;
- a data parsing module (DPM) configured to query the one or more local databases for a set of medical data sets and a set of associated medical reports, the medical data sets each comprising at least one medical finding;
- a reference data generation module (RDGM) configured to generate indications of at least a part of the medical findings by applying the natural language processing algorithm to the medical reports;
- a data gating module (DTM) configured to determine whether to allow a local data item comprising one of the medical data sets and a corresponding indication to be incorporated into a training process of the machine learning algorithm;
- a training module (TM) configured to update the machine learned algorithm based on the allowed local data items by
- applying (S61) the machine learning algorithm to the medical data set so as to extract a medical finding from the medical data set corresponding to the at least one medical finding;
- comparing (S62) the extracted medical finding to the at least one indication of the at least one medical finding;
- updating the machine learning algorithm based on the step of comparing; and
- determining (S63) a correspondence level between the extracted medical finding and the at least one indication; wherein
- the machine learning algorithm is only updated if the correspondence level is above a predetermined threshold.

## Patentansprüche

1. Verfahren zum Bereitstellen eines aktualisierten Maschinenlernalgorithmus, umfassend die folgenden Schritte:
- Bereitstellen (S10) eines medizinischen Datensatzes (MDS), umfassend mindestens einen medizinischen Befund;
- Abrufen (S20) eines medizinischen Berichts (MR), der dem medizinischen Datensatz zugeordnet ist, aus einer Datenbank (DB-R), wobei der medizinische Bericht mindestens eine Angabe des mindestens einen medizinischen Befunds umfasst;
- Erhalten (S30) eines Algorithmus zur Verarbeitung natürlicher Sprache (NLPA), der dazu ausgelegt ist, Angaben zu medizinischen Befunden aus strukturiertem und/oder unstrukturiertem Text zu extrahieren;
- Anwenden (S40) des Algorithmus zur Verarbeitung natürlicher Sprache auf den medizinischen Bericht, um die mindestens eine Angabe (IND) des mindestens einen medizinischen Befunds zu extrahieren;
- Erhalten (S50) eines Maschinenlernalgorithmus (MLA), der dazu ausgelegt ist, medizinische Befunde (MF) aus medizinischen Datensätzen zu extrahieren;
- Aktualisieren (S60) des Maschinenlernalgorithmus basierend auf dem medizinischen Datensatz und der extrahierten mindestens einen Angabe; und
- Bereitstellen (S70) des aktualisierten Maschinenlernalgorithmus, wobei der Schritt des Aktualisierens (S60) umfasst:
- Anwenden (S61) des Maschinenlernalgorithmus auf den medizinischen Datensatz, um einen medizinischen Befund aus dem medizinischen Datensatz zu extrahieren, der dem mindestens einen medizinischen Befund entspricht;
- Vergleichen (S62) des extrahierten medizinischen Befunds mit der mindestens einen Angabe des mindestens einen medizinischen Befunds;
- Aktualisieren des Maschinenlernalgorithmus basierend auf dem Schritt des Vergleichens;
- Bestimmen (S63) eines Übereinstimmungsgrads zwischen dem extrahierten medizinischen Befund und der mindestens einen Angabe; wobei
- der Maschinenlernalgorithmus nur aktualisiert wird, wenn der Übereinstimmungsgrad über einem vorbestimmten Schwellenwert liegt.

2. Verfahren nach Anspruch 1, wobei der Übereinstimmungsgrad ein Maß für die Differenz zwischen dem extrahierten medizinischen Befund und der Angabe umfasst.

3. Verfahren nach einem der vorhergehenden Ansprüche, wobei
- der medizinische Datensatz einen medizinischen Bilddatensatz umfasst;
- der medizinische Befund ein Bildmerkmal betrifft, insbesondere eine Läsion, das in dem medizinischen Bilddatensatz dargestellt ist;
- die Angabe einen Typ und/oder eine Position des Bildmerkmals in dem medizinischen Bilddatensatz umfasst; und
- der Maschinenlernalgorithmus dazu ausgelegt ist, Bildmerkmale aus medizinischen Bilddatensätzen als medizinische Befunde zu extrahieren.

4. Verfahren nach Anspruch 3, wobei der Schritt des Aktualisierens umfasst:
Vergleichen (S63) der Angabe und des extrahierten medizinischen Befunds, um zu prüfen, ob die beiden zumindest insofern miteinander in Beziehung stehen, dass sie sich an ungefähr denselben Positionen befinden.

5. Verfahren nach einem der vorhergehenden Ansprüche, wobei der Algorithmus zur Verarbeitung natürlicher Sprache auf einem Transformermodell basiert; und/oder
der Maschinenlernalgorithmus auf einem neuronalen Faltungsnetzwerk basiert.

6. Lokales Modellaktualisierungsmodul (CCD), umfassend:
- eine Schnittstelle (INT), die dazu ausgelegt ist:
einen von einer Modellaggregationsvorrichtung (MAD) bereitgestellten Maschinenlernalgorithmus zu erhalten, wobei der Maschinenlernalgorithmus dazu ausgelegt ist, medizinische Befunde aus medizinischen Datensätzen zu extrahieren;
einen von der Modellaggregationsvorrichtung bereitgestellten Algorithmus zur Verarbeitung natürlicher Sprache zu erhalten,
wobei der Algorithmus zur Verarbeitung natürlicher Sprache dazu ausgelegt ist, Angaben zu medizinischen Befunden aus Textdaten zu extrahieren;
medizinische Datensätze und zugehörige medizinische Berichte aus einer oder mehreren lokalen Datenbanken zu erhalten;
- ein Datenanalysemodul (DPM), das dazu ausgelegt ist, die eine oder mehreren lokalen Datenbanken abzufragen, um eine Menge von medizinischen Datensätzen und eine Menge zugehöriger medizinischer Berichte zu erhalten, wobei die medizinischen Datensätze jeweils mindestens einen medizinischen Befund umfassen;
- ein Referenzdatengenerierungsmodul (RDGM), das dazu ausgelegt ist, Angaben zu zumindest einem Teil der medizinischen Befunde zu erzeugen, indem der Algorithmus zur Verarbeitung natürlicher Sprache auf die medizinischen Berichte angewendet wird;
- ein Daten-Gating-Modul (DTM), das dazu ausgelegt ist, zu bestimmen, ob ein lokales Datenelement, das einen der medizinischen Datensätze und eine entsprechende Angabe umfasst, in einen Trainingsprozess des Maschinenlernalgorithmus aufgenommen werden darf;
- ein Trainingsmodul (TM), das dazu ausgelegt ist, den Maschinenlernalgorithmus basierend auf den zugelassenen lokalen Datenelementen zu aktualisieren durch
- Anwenden (S61) des Maschinenlernalgorithmus auf den medizinischen Datensatz, um einen medizinischen Befund aus dem medizinischen Datensatz zu extrahieren, der dem mindestens einen medizinischen Befund entspricht;
- Vergleichen (S62) des extrahierten medizinischen Befunds mit der mindestens einen Angabe des mindestens einen medizinischen Befunds;
- Aktualisieren des Maschinenlernalgorithmus basierend auf dem Schritt des Vergleichens; und
- Bestimmen (S63) eines Übereinstimmungsgrads zwischen dem extrahierten medizinischen Befund und der mindestens einen Angabe; wobei
- der Maschinenlernalgorithmus nur aktualisiert wird, wenn der Übereinstimmungsgrad über einem vorbestimmten Schwellenwert liegt.

## Revendications

1. Procédé pour donner un algorithme d'apprentissage automatique mis à jour comprenant les stades suivants :
- se procurer (S10) un ensemble (MDS) de données médicales comprenant au moins une observation médicale ;
- rechercher (S20), dans une base (DB-R) de données, un rapport (MR) médical associé à l'ensemble de données médicales, le rapport médical comprenant au moins une indication de la au moins une observation médicale ;
- obtenir (S30) un algorithme (NLPA) de traitement en langage naturel configuré pour extraire des indications d'observations médicales d'un texte structuré et/ou non structuré ;
- appliquer (S40) l'algorithme de traitement en langage naturel au rapport médical, de manière à extraire la au moins une indication (IND) de la au moins une observation médicale ;
- obtenir (S50) un algorithme (MLA) d'apprentissage automatique configuré pour extraire des observations (MF) médicales d'ensembles de données médicales ;
- mettre (S60) à jour l'algorithme d'apprentissage automatique sur la base d'un ensemble de données médicales et de la au moins une indication extraite ; et
- donner (S70) l'algorithme d'apprentissage automatique mis à jour, dans lequel le stade de mise (S60) à jour comprend :
- appliquer (S61) l'algorithme d'apprentissage automatique à l'ensemble de données médicales, de manière à extraire une observation médicale de l'ensemble de données médicales correspondant à la au moins une observation médicale ;
- comparer (S62) l'observation médicale extraite à la au moins une indication de la au moins une observation médicale ;
- mettre à jour l'algorithme d'apprentissage automatique sur la base du stade de comparaison,
- déterminer (S63) un niveau de correspondance entre l'observation médicale extraite et la au moins une indication ; dans lequel
- l'algorithme d'apprentissage automatique est mis à jour seulement si le niveau de correspondance est au-dessus d'un seuil déterminé à l'avance.

2. Procédé suivant la revendication 1, dans lequel le niveau de correspondance comprend une mesure de différence entre l'observation médicale extraite et l'indication.

3. Procédé suivant l'une quelconque des revendications précédentes, dans lequel
- l'ensemble de données médicales comprend un ensemble de données d'image médicale ;
- l'observation médicale se rapporte à une caractéristique d'images, en particulier à une lésion, dépeinte dans l'ensemble de données d'image médicale ;
- l'indication comprend un type et/ou un emplacement de la caractéristique d'image dans l'ensemble de données d'image médicale ; et
- l'algorithme d'apprentissage automatique est configuré pour extraire des caractéristiques d'image d'ensembles de données d'image médicale en tant qu'observations médicales.

4. Procédé suivant la revendication 3, dans lequel le stade de mise à jour comprend :
comparer (S63) l'indication et l'observation médicale extraite pour vérifier si les deux sont au moins en relation dans le sens où elles sont disposées à peu près aux mêmes endroits.

5. Procédé suivant l'une quelconque des revendications précédentes, dans lequel
l'algorithme de traitement en langage naturel repose sur un modèle de transformateur ; et/ou
l'algorithme d'apprentissage automatique repose sur un réseau neuronal de convolution.

6. Module (CCD) local de mise à jour de modèle, comprenant :
- une interface (INT) configurée pour :
obtenir, d'un dispositif (MAD) agrégateur de modèle, un algorithme d'apprentissage automatique, l'algorithme d'apprentissage automatique étant configuré pour extraire des observations médicales d'ensembles de données médicales ;
obtenir, du dispositif agrégateur de modèle, un algorithme de traitement en langage naturel, l'algorithme de traitement en langage naturel étant configuré pour extraire des indications d'observations médicales de données de texte ;
obtenir, d'une ou plusieurs bases locales de données, des ensembles de données médicales et des rapports médicaux associés ;
- un module (DPM) découpeur de données configuré pour consulter la une ou les plusieurs bases locales de données pour un ensemble d'ensembles de données médicales et un ensemble de rapports médicaux associés, les ensembles de données médicales comprenant chacun au moins une observation médicale ;
- un module (RDGM) de référence de création de données configuré pour créer des indications d'au moins une partie des observations médicales en appliquant l'algorithme de traitement en langage naturel aux rapports médicaux ;
- un module (DTM) donnant accès aux données configuré pour déterminer s'il convient d'allouer un élément d'information locale de données comprenant l'un des ensembles de données médicales et une indication correspondante à incorporer dans une opération d'entraînement de l'algorithme d'apprentissage automatique ;
- un module (TM) d'entraînement configuré pour mettre à jour l'algorithme d'apprentissage automatique sur la base des éléments d'informations locaux de données alloués en
- appliquant (S61) l'algorithme d'apprentissage automatique à l'ensemble de données médicales, de manière à extraire une observation médicale de l'ensemble de données médicales correspondant à la au moins une observation médicale ;
- comparer (S62) l'observation médicale extraite à la au moins une indication de la au moins une observation médicale ;
- mettre à jour l'algorithme d'apprentissage automatique sur la base du stade de comparaison ; et
- déterminer (S63) un niveau correspondant entre l'observation médicale extraite et la au moins une indication ;
dans lequel
- l'algorithme d'apprentissage automatique est mis à jour seulement si le niveau de correspondance est au-dessus d'un seuil déterminé à l'avance.
